# EUROPEAN PATENT APPLICATION

(11) **EP 1 787 996 A2**
(43) Date of publication of application: **23.05.2007**
(21) Application number: 06022358.3
(22) Date of filing: 25.10.2006
(51) Int. Cl.: C07K 14/705, G01N 30/00

(54) **Cancer-suppressing agents**

(30) Priority: 25.10.2005 JP 2005309921
(71) Applicant: Fujifilm Corporation, Minato-Ku Tokyo 106-8620 (JP); Tokyo Medical and Dental University, Tokyo 113-8510 (JP)
(72) Inventor: Misawa, Akiko, Bunkyo-ku Tokyo 113-8510 (JP); Inoue, Jun, Bunkyo-ku Tokyo 113-8510 (JP); Imoto, Issei, Bunkyo-ku Tokyo 113-8510 (JP); Inazawa, Johji, Bunkyo-ku Tokyo 113-8510 (JP)
(74) Representative: Albrecht, Thomas

(57) **Abstract**

It is an object of the present invention to provide a cancer-suppressing agent comprising a novel cancer-suppressing gene based on the discovery of such cancer-suppressing gene. The present invention provides a cancer-suppressing agent which comprises NR1I2 gene or a homologous gene thereof; and a cancer-suppressing agent which comprises NR1I2 protein or a homologous protein thereof.

### Annex to the application documents - subsequently filed sequences listing

SEQUENCE LISTING

<110> Fuji Photo Film Co.

<120> Cancer suppressing agent

<130> 17095EP

<160> 2

<170> PatentIn version 3.3

<210> 1

<211> 2146

<212> DNA

<213> Homo sapiens

<400> 1

tgaaatatag gtgagagaca agattgtctc atatccgggg aaatcataac ctatgactag 60

gacgggaaga ggaagcactg cctttacttc agtgggaatc tcggcctcag cctgcaagcc 120

aagtgttcac agtgagaaaa gcaagagaat aagctaatac tcctgtcctg aacaaggcag 180

cggctccttg gtaaagctac tccttgatcg atcctttgca ccggattgtt caaagtggac 240

cccaggggag aagtcggagc aaagaactta ccaccaagca gtccaagagg cccagaagca 300

aacctggagg tgagacccaa agaaagctgg aaccatgctg actttgtaca ctgtgaggac 360

acagagtctg ttcctggaaa gcccagtgtc aacgcagatg aggaagtcgg aggtccccaa 420

atctgccgtg tatgtgggga caaggccact ggctatcact tcaatgtcat gacatgtgaa 480

ggatgcaagg gctttttcag gagggccatg aaacgcaacg cccggctgag gtgccccttc 540

cggaagggcg cctgcgagat cacccggaag acccggcgac agtgccaggc ctgccgcctg 600

cgcaagtgcc tggagagcgg catgaagaag gagatgatca tgtccgacga ggccgtggag 660

gagaggcggg ccttgatcaa gcggaagaaa agtgaacgga cagggactca gccactggga 720

gtgcaggggc tgacagagga gcagcggatg atgatcaggg agctgatgga cgctcagatg 780

aaaacctttg acactacctt ctcccatttc aagaatttcc ggctgccagg ggtgcttagc 840

agtggctgcg agttgccaga gtctctgcag gccccatcga gggaagaagc tgccaagtgg 900

agccaggtcc ggaaagatct gtgctctttg aaggtctctc tgcagctgcg gggggaggat 960

ggcagtgtct ggaactacaa acccccagcc gacagtggcg ggaaagagat cttctccctg 1020

ctgccccaca tggctgacat gtcaacctac atgttcaaag gcatcatcag ctttgccaaa 1080

gtcatctcct acttcaggga cttgcccatc gaggaccaga tctccctgct gaagggggcc 1140

gctttcgagc tgtgtcaact gagattcaac acagtgttca acgcggagac tggaacctgg 1200
gagtgtggcc ggctgtccta ctgcttggaa gacactgcag gtggcttcca gcaacttcta 1260
ctggagccca tgctgaaatt ccactacatg ctgaagaagc tgcagctgca tgaggaggag 1320
tatgtgctga tgcaggccat ctccctcttc tccccagacc gcccaggtgt gctgcagcac 1380
cgcgtggtgg accagctgca ggagcaattc gccattactc tgaagtccta cattgaatgc 1440
aatcggcccc agcctgctca taggttcttg ttcctgaaga tcatggctat gctcaccgag 1500
ctccgcagca tcaatgctca gcacacccag cggctgctgc gcatccagga catacacccc 1560
tttgctacgc ccctcatgca ggagttgttc ggcatcacag gtagctgagc ggctgccctt 1620
gggtgacacc tccgagaggc agccagaccc agagccctct gagccgccac tcccgggcca 1680
agacagatgg acactgccaa gagccgacaa tgccctgctg gcctgtctcc ctagggaatt 1740
cctgctatga cagctggcta gcattcctca ggaaggacat gggtgccccc cacccccagt 1800
tcagtctgta gggagtgaag ccacagactc ttacgtggag agtgcactga cctgtaggtc 1860
aggaccatca gagaggcaag gttgcccttt ccttttaaaa ggccctgtgg tctggggaga 1920
aatccctcag atcccactaa agtgtcaagg tgtggaaggg accaagcgac caaggatagg 1980
ccatctgggg tctatgccca catacccacg tttgttcgct tcctgagtct tttcattgct 2040
acctctaata gtcctgtctc ccacttccca ctcgttcccc tcctcttccg agctgctttg 2100
tgggctccag gcctgtactc atcggcaggt gcatgagtat ctgtgg 2146
<210> 2
<211> 434
<212> PRT
<213> Homo sapiens
<400> 2
Met Glu Val Arg Pro Lys Glu Ser Trp Asn His Ala Asp Phe Val His
1               5                   10                  15
Cys Glu Asp Thr Glu Ser Val Pro Gly Lys Pro Ser Val Asn Ala Asp
            20                  25                  30
Glu Glu Val Gly Gly Pro Gln Ile Cys Arg Val Cys Gly Asp Lys Ala
        35                  40                  45
Thr Gly Tyr His Phe Asn Val Met Thr Cys Glu Gly Cys Lys Gly Phe
    50                  55                  60

Phe Arg Arg Ala Met Lys Arg Asn Ala Arg Leu Arg Cys Pro Phe Arg
65                  70                  75                  80
Lys Gly Ala Cys Glu Ile Thr Arg Lys Thr Arg Arg Gln Cys Gln Ala
                85                  90                  95
Cys Arg Leu Arg Lys Cys Leu Glu Ser Gly Met Lys Lys Glu Met Ile
            100                 105                 110
Met Ser Asp Glu Ala Val Glu Glu Arg Arg Ala Leu Ile Lys Arg Lys
        115                 120                 125
Lys Ser Glu Arg Thr Gly Thr Gln Pro Leu Gly Val Gln Gly Leu Thr
    130                 135                 140
Glu Glu Gln Arg Met Met Ile Arg Glu Leu Met Asp Ala Gln Met Lys
145                 150                 155                 160
Thr Phe Asp Thr Thr Phe Ser His Phe Lys Asn Phe Arg Leu Pro Gly
                165                 170                 175
Val Leu Ser Ser Gly Cys Glu Leu Pro Glu Ser Leu Gln Ala Pro Ser
            180                 185                 190
Arg Glu Glu Ala Ala Lys Trp Ser Gln Val Arg Lys Asp Leu Cys Ser
        195                 200                 205
Leu Lys Val Ser Leu Gln Leu Arg Gly Glu Asp Gly Ser Val Trp Asn
    210                 215                 220
Tyr Lys Pro Pro Ala Asp Ser Gly Gly Lys Glu Ile Phe Ser Leu Leu
225                 230                 235                 240
Pro His Met Ala Asp Met Ser Thr Tyr Met Phe Lys Gly Ile Ile Ser
                245                 250                 255
Phe Ala Lys Val Ile Ser Tyr Phe Arg Asp Leu Pro Ile Glu Asp Gln
            260                 265                 270
Ile Ser Leu Leu Lys Gly Ala Ala Phe Glu Leu Cys Gln Leu Arg Phe
        275                 280                 285
Asn Thr Val Phe Asn Ala Glu Thr Gly Thr Trp Glu Cys Gly Arg Leu
    290                 295                 300

Ser Tyr Cys Leu Glu Asp Thr Ala Gly Gly Phe Gln Gln Leu Leu Leu
305                 310                 315                 320
Glu Pro Met Leu Lys Phe His Tyr Met Leu Lys Lys Leu Gln Leu His
                325                 330                 335
Glu Glu Glu Tyr Val Leu Met Gln Ala Ile Ser Leu Phe Ser Pro Asp
            340                 345                 350
Arg Pro Gly Val Leu Gln His Arg Val Val Asp Gln Leu Gln Glu Gln
        355                 360                 365
Phe Ala Ile Thr Leu Lys Ser Tyr Ile Glu Cys Asn Arg Pro Gln Pro
    370                 375                 380
Ala His Arg Phe Leu Phe Leu Lys Ile Met Ala Met Leu Thr Glu Leu
385                 390                 395                 400
Arg Ser Ile Asn Ala Gln His Thr Gln Arg Leu Leu Arg Ile Gln Asp
                405                 410                 415
Ile His Pro Phe Ala Thr Pro Leu Met Gln Glu Leu Phe Gly Ile Thr
            420                 425                 430
Gly Ser

SEQUENCE LISTING

<110> Fujifilm Corporation
      Tokyo Medical and Dental University

<120> Cancer-suppressing agents

<130> 17095EP

<140> 06 022 358.3
<141> 2006-10-25

<150> JP 309921/2005
<151> 2005-10-25

<160> 2

<170> PatentIn version 3.3

<210> 1
<211> 2146
<212> DNA
<213> Homo sapiens


<220>
<221> CDS
<222> (304)..(1608)
<223> First amino acid is Met according to CDS feature of AF061056 GenBank
accession number

<400> 1
tgaaatatag gtgagagaca agattgtctc atatccgggg aaatcataac ctatgactag      60

gacgggaaga ggaagcactg cctttacttc agtgggaatc tcggcctcag cctgcaagcc     120

aagtgttcac agtgagaaaa gcaagagaat aagctaatac tcctgtcctg aacaaggcag     180

cggctccttg gtaaagctac tccttgatcg atcctttgca ccggattgtt caaagtggac     240

cccaggggag aagtcggagc aaagaactta ccaccaagca gtccaagagg cccagaagca     300

aac ctg gag gtg aga ccc aaa gaa agc tgg aac cat gct gac ttt gta       348
    Leu Glu Val Arg Pro Lys Glu Ser Trp Asn His Ala Asp Phe Val
    1               5                   10                  15

cac tgt gag gac aca gag tct gtt cct gga aag ccc agt gtc aac gca       396
His Cys Glu Asp Thr Glu Ser Val Pro Gly Lys Pro Ser Val Asn Ala
                20                  25                  30

gat gag gaa gtc gga ggt ccc caa atc tgc cgt gta tgt ggg gac aag       444
Asp Glu Glu Val Gly Gly Pro Gln Ile Cys Arg Val Cys Gly Asp Lys
            35                  40                  45

gcc act ggc tat cac ttc aat gtc atg aca tgt gaa gga tgc aag ggc       492
Ala Thr Gly Tyr His Phe Asn Val Met Thr Cys Glu Gly Cys Lys Gly
        50                  55                  60

ttt ttc agg agg gcc atg aaa cgc aac gcc cgg ctg agg tgc ccc ttc 540
Phe Phe Arg Arg Ala Met Lys Arg Asn Ala Arg Leu Arg Cys Pro Phe
    65                  70                  75

cgg aag ggc gcc tgc gag atc acc cgg aag acc cgg cga cag tgc cag 588
Arg Lys Gly Ala Cys Glu Ile Thr Arg Lys Thr Arg Arg Gln Cys Gln
80                  85                  90                  95

gcc tgc cgc ctg cgc aag tgc ctg gag agc ggc atg aag aag gag atg 636
Ala Cys Arg Leu Arg Lys Cys Leu Glu Ser Gly Met Lys Lys Glu Met
                100                 105                 110

atc atg tcc gac gag gcc gtg gag gag agg cgg gcc ttg atc aag cgg 684
Ile Met Ser Asp Glu Ala Val Glu Glu Arg Arg Ala Leu Ile Lys Arg
            115                 120                 125

aag aaa agt gaa cgg aca ggg act cag cca ctg gga gtg cag ggg ctg 732
Lys Lys Ser Glu Arg Thr Gly Thr Gln Pro Leu Gly Val Gln Gly Leu
        130                 135                 140

aca gag gag cag cgg atg atg atc agg gag ctg atg gac gct cag atg 780
Thr Glu Glu Gln Arg Met Met Ile Arg Glu Leu Met Asp Ala Gln Met
    145                 150                 155

aaa acc ttt gac act acc ttc tcc cat ttc aag aat ttc cgg ctg cca 828
Lys Thr Phe Asp Thr Thr Phe Ser His Phe Lys Asn Phe Arg Leu Pro
160                 165                 170                 175

ggg gtg ctt agc agt ggc tgc gag ttg cca gag tct ctg cag gcc cca 876
Gly Val Leu Ser Ser Gly Cys Glu Leu Pro Glu Ser Leu Gln Ala Pro
                180                 185                 190

tcg agg gaa gaa gct gcc aag tgg agc cag gtc cgg aaa gat ctg tgc 924
Ser Arg Glu Glu Ala Ala Lys Trp Ser Gln Val Arg Lys Asp Leu Cys
            195                 200                 205

tct ttg aag gtc tct ctg cag ctg cgg ggg gag gat ggc agt gtc tgg 972
Ser Leu Lys Val Ser Leu Gln Leu Arg Gly Glu Asp Gly Ser Val Trp
        210                 215                 220

aac tac aaa ccc cca gcc gac agt ggc ggg aaa gag atc ttc tcc ctg 1020
Asn Tyr Lys Pro Pro Ala Asp Ser Gly Gly Lys Glu Ile Phe Ser Leu
    225                 230                 235

ctg ccc cac atg gct gac atg tca acc tac atg ttc aaa ggc atc atc 1068
Leu Pro His Met Ala Asp Met Ser Thr Tyr Met Phe Lys Gly Ile Ile
240                 245                 250                 255

agc ttt gcc aaa gtc atc tcc tac ttc agg gac ttg ccc atc gag gac 1116
Ser Phe Ala Lys Val Ile Ser Tyr Phe Arg Asp Leu Pro Ile Glu Asp
                260                 265                 270

cag atc tcc ctg ctg aag ggg gcc gct ttc gag ctg tgt caa ctg aga 1164
Gln Ile Ser Leu Leu Lys Gly Ala Ala Phe Glu Leu Cys Gln Leu Arg
            275                 280                 285

ttc aac aca gtg ttc aac gcg gag act gga acc tgg gag tgt ggc cgg 1212
Phe Asn Thr Val Phe Asn Ala Glu Thr Gly Thr Trp Glu Cys Gly Arg
        290                 295                 300

ctg tcc tac tgc ttg gaa gac act gca ggt ggc ttc cag caa ctt cta 1260
Leu Ser Tyr Cys Leu Glu Asp Thr Ala Gly Gly Phe Gln Gln Leu Leu
    305                 310                 315

ctg gag ccc atg ctg aaa ttc cac tac atg ctg aag aag ctg cag ctg 1308
Leu Glu Pro Met Leu Lys Phe His Tyr Met Leu Lys Lys Leu Gln Leu
320                 325                 330                 335

cat gag gag gag tat gtg ctg atg cag gcc atc tcc ctc ttc tcc cca 1356
His Glu Glu Glu Tyr Val Leu Met Gln Ala Ile Ser Leu Phe Ser Pro
                340                 345                 350

gac cgc cca ggt gtg ctg cag cac cgc gtg gtg gac cag ctg cag gag 1404
Asp Arg Pro Gly Val Leu Gln His Arg Val Val Asp Gln Leu Gln Glu
            355                 360                 365

caa ttc gcc att act ctg aag tcc tac att gaa tgc aat cgg ccc cag 1452
Gln Phe Ala Ile Thr Leu Lys Ser Tyr Ile Glu Cys Asn Arg Pro Gln
        370                 375                 380

cct gct cat agg ttc ttg ttc ctg aag atc atg gct atg ctc acc gag 1500
Pro Ala His Arg Phe Leu Phe Leu Lys Ile Met Ala Met Leu Thr Glu
    385                 390                 395

ctc cgc agc atc aat gct cag cac acc cag cgg ctg ctg cgc atc cag 1548
Leu Arg Ser Ile Asn Ala Gln His Thr Gln Arg Leu Leu Arg Ile Gln
400                 405                 410                 415

gac ata cac ccc ttt gct acg ccc ctc atg cag gag ttg ttc ggc atc 1596
Asp Ile His Pro Phe Ala Thr Pro Leu Met Gln Glu Leu Phe Gly Ile
                420                 425                 430

aca ggt agc tga gcggctgccc ttgggtgaca cctccgagag gcagccagac 1648
Thr Gly Ser

ccagagccct ctgagccgcc actcccgggc caagacagat ggacactgcc aagagccgac 1708

aatgccctgc tggcctgtct ccctagggaa ttcctgctat gacagctggc tagcattcct 1768

caggaaggac atgggtgccc cccaccccca gttcagtctg tagggagtga agccacagac 1828

tcttacgtgg agagtgcact gacctgtagg tcaggaccat cagagaggca aggttgccct 1888

ttccttttaa aaggccctgt ggtctgggga gaaatccctc agatcccact aaagtgtcaa 1948

ggtgtggaag ggaccaagcg accaaggata ggccatctgg ggtctatgcc cacataccca 2008

cgtttgttcg cttcctgagt cttttcattg ctacctctaa tagtcctgtc tcccacttcc 2068

cactcgttcc cctcctcttc cgagctgctt tgtgggctcc aggcctgtac tcatcggcag 2128

gtgcatgagt atctgtgg 2146

<210>  2
<211>  434
<212>  PRT
<213>  Homo sapiens

<220>
<223>  First amino acid is Met according to AAD05436 Genpept accession number

<400>  2

Leu Glu Val Arg Pro Lys Glu Ser Trp Asn His Ala Asp Phe Val His
1               5                   10                  15

Cys Glu Asp Thr Glu Ser Val Pro Gly Lys Pro Ser Val Asn Ala Asp
            20                  25                  30

Glu Glu Val Gly Gly Pro Gln Ile Cys Arg Val Cys Gly Asp Lys Ala
        35                  40                  45

Thr Gly Tyr His Phe Asn Val Met Thr Cys Glu Gly Cys Lys Gly Phe
    50                  55                  60

Phe Arg Arg Ala Met Lys Arg Asn Ala Arg Leu Arg Cys Pro Phe Arg
65                  70                  75                  80

Lys Gly Ala Cys Glu Ile Thr Arg Lys Thr Arg Arg Gln Cys Gln Ala
                85                  90                  95

Cys Arg Leu Arg Lys Cys Leu Glu Ser Gly Met Lys Lys Glu Met Ile
            100                 105                 110

Met Ser Asp Glu Ala Val Glu Glu Arg Arg Ala Leu Ile Lys Arg Lys
        115                 120                 125

Lys Ser Glu Arg Thr Gly Thr Gln Pro Leu Gly Val Gln Gly Leu Thr
    130                 135                 140

Glu Glu Gln Arg Met Met Ile Arg Glu Leu Met Asp Ala Gln Met Lys
145                 150                 155                 160

Thr Phe Asp Thr Thr Phe Ser His Phe Lys Asn Phe Arg Leu Pro Gly
                165                 170                 175

Val Leu Ser Ser Gly Cys Glu Leu Pro Glu Ser Leu Gln Ala Pro Ser
            180                 185                 190

Arg Glu Glu Ala Ala Lys Trp Ser Gln Val Arg Lys Asp Leu Cys Ser
        195                 200                 205

Leu Lys Val Ser Leu Gln Leu Arg Gly Glu Asp Gly Ser Val Trp Asn
    210                 215                 220

Tyr Lys Pro Pro Ala Asp Ser Gly Gly Lys Glu Ile Phe Ser Leu Leu
225                 230                 235                 240

Pro His Met Ala Asp Met Ser Thr Tyr Met Phe Lys Gly Ile Ile Ser
                245                 250                 255

Phe Ala Lys Val Ile Ser Tyr Phe Arg Asp Leu Pro Ile Glu Asp Gln
            260                 265                 270

Ile Ser Leu Leu Lys Gly Ala Ala Phe Glu Leu Cys Gln Leu Arg Phe
        275                 280                 285

Asn Thr Val Phe Asn Ala Glu Thr Gly Thr Trp Glu Cys Gly Arg Leu
    290                 295                 300

Ser Tyr Cys Leu Glu Asp Thr Ala Gly Gly Phe Gln Gln Leu Leu Leu
305                 310                 315                 320

Glu Pro Met Leu Lys Phe His Tyr Met Leu Lys Lys Leu Gln Leu His
                325                 330                 335

Glu Glu Glu Tyr Val Leu Met Gln Ala Ile Ser Leu Phe Ser Pro Asp
            340                 345                 350

Arg Pro Gly Val Leu Gln His Arg Val Val Asp Gln Leu Gln Glu Gln
        355                 360                 365

Phe Ala Ile Thr Leu Lys Ser Tyr Ile Glu Cys Asn Arg Pro Gln Pro
    370                 375                 380

Ala His Arg Phe Leu Phe Leu Lys Ile Met Ala Met Leu Thr Glu Leu
385                 390                 395                 400

Arg Ser Ile Asn Ala Gln His Thr Gln Arg Leu Leu Arg Ile Gln Asp
                405                 410                 415

Ile His Pro Phe Ala Thr Pro Leu Met Gln Glu Leu Phe Gly Ile Thr
            420                 425                 430

Gly Ser

## Description

### Technical Field

The present invention relates to medical uses of a cancer-suppressing gene and a protein encoded by the same.

### Background Art

It has been known that onset of cancer is induced by mutation or quantitative change of a cell protein. Along with recent development in genetic engineering, it has become possible to amplify a gene encoding a specific protein and to analyze gene mutation in cancer cells, resulting in breakthroughs in the field of cancer research. Hitherto, analysis and identification of oncogenes involved in the canceration of cells and the abnormal growth of cancer cells have made progress. Meanwhile, in recent years, cancer-suppressing genes have been gaining attention. Mutation or the decreased expression level of cancer-suppressing gene leads to canceration of cells. Examples of cancer-suppressing genes that have been identified include Rb gene of retinoblastoma, p53 gene and APC gene of large-bowel cancer, and WT1 gene of Wilms tumor. For instance, an example of a cancer-suppressing agent that uses WT1 gene has been reported (WO2003/002142).

In addition, it has been gradually revealed that cancer development, malignant progression, and metastasis are caused by abnormalities of not only a single gene but also a plurality of genes. In addition, a greater number of unidentified oncogenes and cancer-suppressing genes are now believed to exist. There are many genes known to have effects that suppress cancer. In most cases, screening for such genes has been carried out by an approach of visually detecting mutation of a patient's gene via staining of chromosomal DNA (Yasuhide Yamashita, et al., World J Gastroenterol, 11 (33): 5129-5135, 2005) or by a method wherein a region of gene deletion is roughly selected based on LOH (loss of heterozygosity) analysis so that important gene regions are narrowed down (WO01/032859). However, such methods are not sufficient as means of discovering cancer-suppressing genes. This is because a tremendous number of DNA deletion regions are detected, so that narrowing them down into important gene regions is extremely time- and labor-consuming, which has been a drawback.

### Disclosure of the Invention

It is an object of the present invention to provide a cancer-suppressing agent comprising a novel cancer-suppressing gene based on the discovery of such cancer-suppressing gene. Further, it is another object of the present invention to provide a cancer-suppressing agent comprising a protein encoded by the cancer-suppressing gene, and a method for detecting and diagnosing cancer using the cancer-suppressing gene.

In order to attain the above object, the inventors of the present invention have intensively studied to identify genes that differ in terms of the degree of DNA methylation in neuroblastoma cases. Neuroblastoma is malignant tumor of childhood, which is generated from migrating sympathogonia derived from a neural crest cell during the embryonic stage. In some cancer cells, a genomic region (CpG island) dense with CpG sites in the 5' region of a gene (cancer-suppressing gene) inhibiting carcinogenesis is abnormally methylated, although the major part of such region is normally unmethylated. Such abnormal methylation results in inhibition of the expression of messenger RNA. Such methylation has recently been recognized as an important carcinogenic mechanism, in addition to gene mutation. In the case of the present invention, in order to identify methylated DNA in neuroblastoma, genes that have been hypermethylated in cancer were screened by a newly developed BAMCA (BAC array-based MCA) method (Inazawa J., et al., Cancer Sci. 95 (7), 559, 2004). Further, the inventors of the present invention have succeeded in identifying an NR1I2 (nuclear receptor subfamily 1, group I, member 2; also referred to as pregnane X receptor (PXR)) gene by using a combination of COBRA (combined bisulfite restriction analysis) (Toyota M., et al., Cancer Res. 59, 2307, 1999) and RT-PCR, as a gene the degree of DNA methylation of which increases along with neuroblastoma stage progression, resulting in suppression of gene expression. This has led to the completion of the present invention.

Thus, the present invention provides a cancer-suppressing agent which comprises NR1I2 gene or a homologous gene thereof.

Preferably, the gene or a homologous gene thereof is incorporated into a vector.

Preferably, the vector is a viral vector or plasmid vector for expression in animal cell.

Preferably, the viral vector is a retroviral vector, adenoviral vector, adeno-associated viral vector, baculovirus vector, vaccinia vector, or lentiviral vector.

Preferably, the gene or a homologous gene thereof is encapsulated in a liposome.

Another embodiment of the present invention provides a cancer-suppressing agent which comprises NR1I2 protein or a homologous protein thereof.

Further another embodiment of the present invention provides a method for detecting and diagnosing cancer, which comprises a step of analyzing NR1I2 gene in a test sample using DNA or RNA containing NR1I2 gene in its entirety or a part thereof.

Preferably, the analysis involves detection of mutation of the gene or detection of abnormal expression level of the gene.

Preferably, the method for detecting and diagnosing cancer is a method for screening for cancer to which the cancer-suppressing agent of the present invention can be applied.

Further another embodiment of the present invention provides a method for detecting and diagnosing cancer, which comprises a step of analyzing NR1I2 protein in a test sample using an antibody against NR1I2 protein or fragment thereof.

Preferably, the analysis involves detection of abnormal expression level of the protein.

Preferably, the method for detecting and diagnosing cancer is a method for screening for cancer to which the cancer-suppressing agent of the present invention can be applied.

### Brief Description of the Drawings

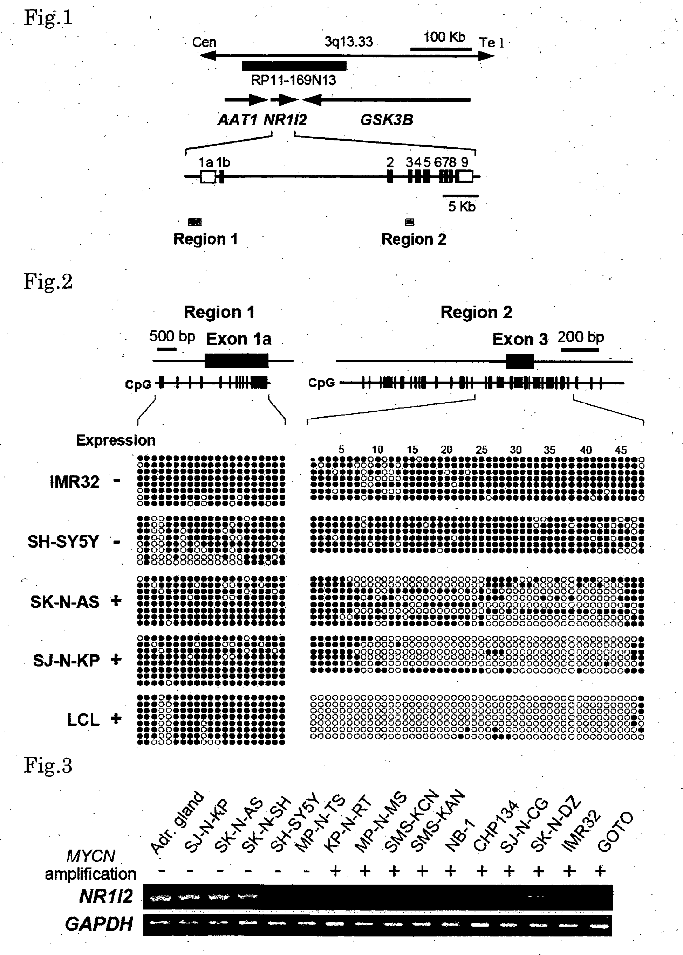

Fig. 1 shows the gene structure of BAC clone 169N13 that is hypermethylated in a neuroblastoma cell line obtained by BAMCA method.
Fig. 2 shows results of sequencing of methylated CpG-rich NR1I2 gene regions (Region 1 and Region 2) that were confirmed in the cases of cell lines regarding which NR1I2 gene expression was known to be either present or absent. Methylated and unmethylated regions are shown in black and gray, respectively.
Fig. 3 shows results of observation of the expression of messenger RNA of NR1I2 gene by RT-PCR and electrophoresis using an adrenal gland (Adr-gland) of a healthy individual and 15 neuroblastoma cell lines cell lines where the amplification of N-myc gene in genomic DNA had been known. As a control for the template amount, GAPDH was shown in the lower column.

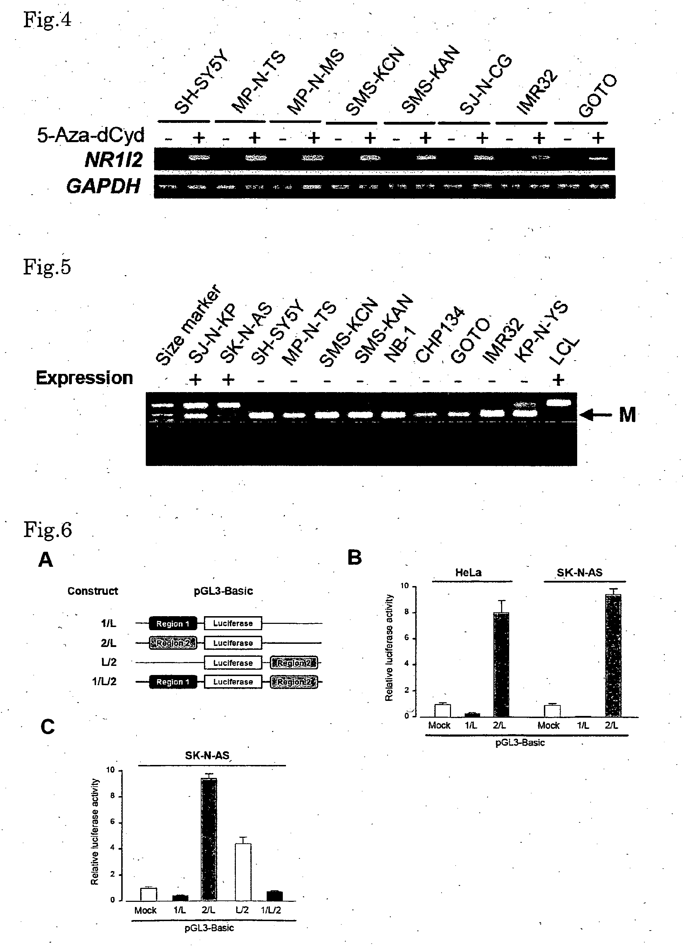

Fig. 4 shows results of observation of the expression of messenger RNA of NR1I2 gene by RT-PCR and electrophoresis using 8 neuroblastoma cell lines subjected to culture in the presence (+) or absence (-) of 5-aza-dCyd, which is an inhibitor of DNA methyltransferase. As a control for the template amount, GAPDH is shown in the lower column.
Fig. 5 shows results of detection of methylation in Region 2 of NR1I2 gene by COBRA method using 11 neuroblastoma cells and a B lymphoma cell line LCL. The results indicate that the cell lines exhibiting band at the M position have a methylated Region 2. A cell line in which messenger RNA expression of NR1I2 gene is present and a cell line in which the same is absent are denoted with "+" and "-" signs, respectively.
Fig. 6A shows the structure of the construct containing Regions 1 and 2 of NR1I2 gene based on reporter assay. Fig. 6B shows results of measurement of luciferase activity in HeLa and SK-N-AS cells using 1/L and 2/L. Mock is a negative control used upon gene introduction of a vector lacking a foreign gene. Fig. 6C shows results of measurement of luciferase activity in Mock and SK-N-AS cells subjected to gene introduction using 1/L, 2/L, L/2, and 1/L/2.

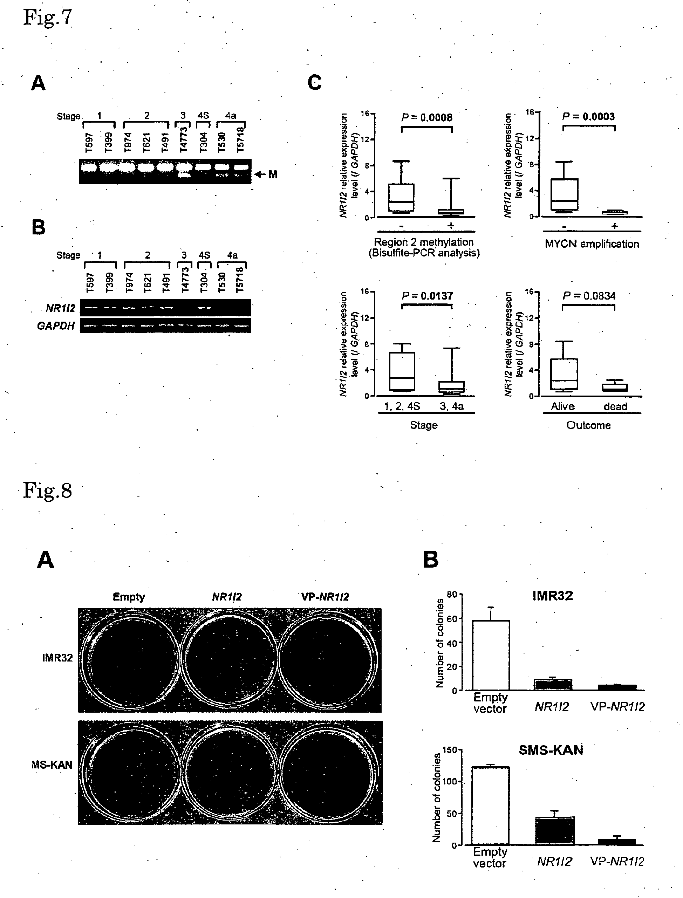

Fig. 7A shows results of detection of methylation in Region 2 of NR1I2 gene by COBRA method using 9 clinical samples subjected to stage classification. The figure shows that samples exhibiting band at the M position have a methylated Region 2. Fig. 7B shows results of RT-PCR that was conducted to confirm the expression of messenger RNA of NR1I2 gene using RNAs obtained from the above 9 samples. As a control for the template amount, GAPDH is shown in the lower column. Fig. 7C shows graphs indicating the presence or absence of methylated Region 2 of NR1I2 gene, the presence or absence of N-myc gene amplification, neuroblastoma stage classification, and prognosis. The graphs were made in a manner reflecting the fact that 47 neuroblastoma samples were subjected to RT-PCR such that the expression level of messenger RNA of NR1I2 gene was measured, followed by correction using GAPDH.
Fig. 8A shows images of culture dishes in which an expression vector alone (Empty), NR1I2 gene, and NR1I2 gene to which VP16 was bound were introduced into neuroblastoma cell lines IMR32 and SMS-KAN, followed by 3-week soft agar culture. Fig. 8B shows graphs indicating the numbers of proliferating cell masses (colonies) that were observed during the experiment of fig. 8A.

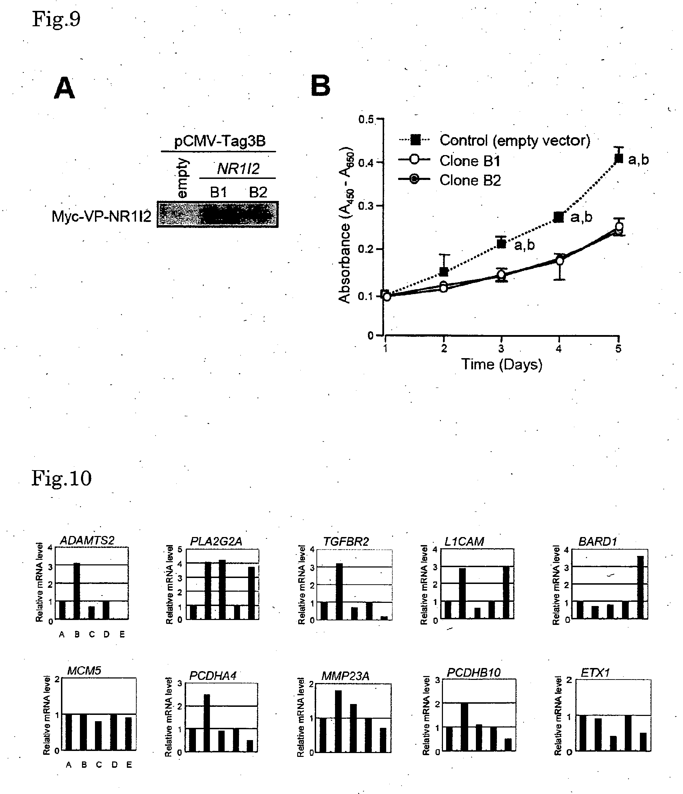

Fig. 9 shows results of detection by Western blotting of disrupted cell solutions of a control cell (empty) established by introducing an expression vector alone into an SMS-KAN cell and two types of cell lines into which the NR1I2 gene had been introduced established by introducing an expression vector and an NR1I2 gene into SMS-KAN cells (B1 and B2), using an antibody against an myc protein serving as a tag sequence. Fig. 9B shows results of observation described in a graph having a vertical axis indicating growth rates of the above 3 types of cells using mitochondrial dehydrogenase activity as an index for number of cells.
Fig. 10 shows results of observation of the expression levels of 10 genes by RT-PCR and electrophoresis. The 10 genes were selected from among those listed in tables 3 and 4 which exhibit 1.5-fold or greater expression levels due to NR1I2 gene expression, as candidate genes involved in cancer growth inhibition. A: SMS-KAN as a control, B: KAN-NR1I2 expression clone B1, C: KAN-NR1I2 expression clone B2, D: GOTO as a control, and E: GOTO-NR1I2 expression clone A1, were used. The GAPDH gene was subjected to a similar experiment, and the results were used as control values for correction. The expression level of each gene was then calculated as a relative value with respect to the control value.

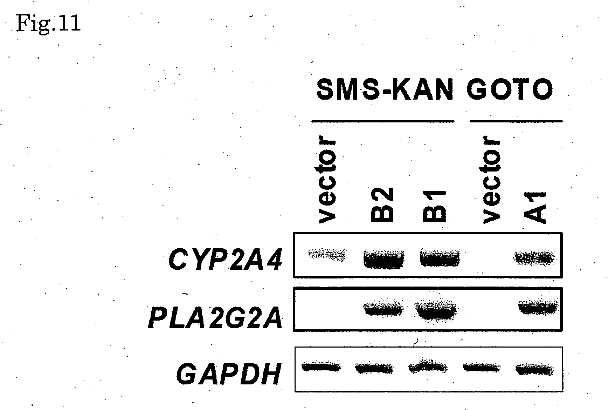

Fig. 11 shows electrophoresis images of CYP2A4 gene, PLA2G2A gene, and GAPDH gene subjected to RT-PCR using a control (vector) in an SMS-KAN cell, NR1I2 expression clones B1 and B2, a control (vector) in GOTO cell, and NR1I2 expression clone A1.

### Best Mode for Carrying Out the Invention

Hereafter, the embodiments and implementation of the present invention will be described in detail.

### (1) Cancer-suppressing agent

In accordance with one embodiment of the present invention, the cancer-suppressing agent of the present invention comprises as an active ingredient NR1I2 gene or a homologous gene thereof. In accordance with another embodiment of the present invention, the cancer-suppressing agent of the present invention comprises as an active ingredient NR1I2 protein or a homologous protein thereof.

The nucleotide sequence of NR1I2 gene and the amino acid sequence of NR1I2 protein have already been known (Lehmann, J. M. et al. J. Clin. Invest. 102, 1016-1023, 1998). The nucleotide sequence of NR1I2 gene has been registered with the database of the National Center for Biotechnology Information (accession no. AF061056). Also, the amino acid sequence of NR1I2 protein has been registered with the same database (accession no. AAD05436.1). The nucleotide sequence of NR1I2 gene is set forth in SEQ ID NO: 1. NR1I2 protein is encoded by the region between positions 304 and 1608 of the nucleotide sequence set forth in SEQ ID NO: 1. The amino acid sequence thereof is set forth in SEQ ID NO: 2.

Herein, the term "NR1I2 gene" refers to a human-derived gene that is specified with the above nucleotide sequence. The term "NR1I2 protein" refers to a protein that is specified with the above amino acid sequence and encoded by NR1I2 gene.

NR1I2 gene may be cDNA obtained from cultured cells using a technique known by persons skilled in the art, or may be synthesized by PCR or the like based on the nucleotide sequence set forth in SEQ ID NO: 1. When DNA having the nucleotide sequence set forth in SEQ ID NO: 1 is obtained by PCR, PCR is carried out using a human chromosomal DNA or a cDNA library as a template and a pair of primers designed to be able to amplify the nucleotide sequence set forth in SEQ ID NO: 1. DNA fragments amplified by PCR can be cloned into an adequate vector that can be amplified in a host such as *Escherichia coli.*

The aforementioned operations, such as probe or primer preparation, cDNA library construction, screening of a cDNA library, and cloning of a target gene, are known to persons skilled in the art. Such operations can be carried out in accordance with methods described in Molecular Cloning: A laboratory Manual, 2nd Ed., Cold Spring Harbor Laboratory, Cold Spring Harbor, NY, 1989, Current Protocols in Molecular Biology, Supplement 1-38, John Wiley & Sons (1987-1997) and the like.

In accordance with the present invention, the term "homologous gene of NR1I2 gene" refers to: a gene having a nucleotide sequence encoding a protein having cancer-suppressing activity, such nucleotide sequence being derived from the nucleotide sequence set forth in SEQ ID NO: 1 by deletion, addition, or substitution of one or several amino acids; or a gene having a nucleotide sequence encoding a protein having cancer-suppressing activity, such nucleotide sequence being hybridized with the nucleotide sequence set forth in SEQ ID NO: 1 under stringent conditions. In addition, a fragment of NR1I2 gene is included in the definition of homologous gene of NR1I2 gene.

Regarding the above "nucleotide sequence derived from the nucleotide sequence set forth in SEQ ID NO: 1 by deletion, addition, or substitution of one or several amino acids," the range of "one to several amino acids" is not particularly limited. For instance, such description indicates 1 to 60 amino acids, preferably 1 to 30 amino acids, more preferably 1 to 20 amino acids, further preferably 1 to 10 amino acids, and particularly preferably 1 to 5 amino acids.

The level of "cancer-suppressing activity" above is not particularly limited. However, preferably, such level of cancer-suppressing activity is substantially equivalent to or higher than cancer-suppressing activity of NR1I2 protein (hereafter, the term "cancer-suppressing activity" herein has the meaning given above).

Thus, as long as the "homologous gene of NR1I2 gene" has the structure and function described above, its origin is not particularly limited. Therefore, it may be derived from mammals excluding humans, or may be obtained by artificially introducing mutation into a gene derived from mammals such as humans. Note that when the gene is used as a cancer-suppressing agent as described below, it is preferable that the gene be derived from humans in view of clinical safety.

The aforementioned "gene having a nucleotide sequence encoding a protein having cancer-suppressing activity, such nucleotide sequence being derived from the nucleotide sequence set forth in SEQ ID NO: 1 by deletion, addition, or substitution of one or several amino acids" can be produced by any methods known by persons skilled in the art such as chemical synthesis, gene engineering techniques, and mutagenesis methods. Specifically, the aforementioned gene can be obtained by utilizing DNA having the nucleotide sequence set forth in SEQ ID NO: 1 and introducing mutation into the DNA. For instance, a method wherein DNA having the nucleotide sequence set forth in SEQ ID NO: 1 is allowed to come into contact with an agent serving as a mutagen, a method of UV irradiation, a gene engineering technique, and the like can be used. Site-directed mutagenesis is one of gene engineering techniques. It is useful because a specific mutation can be introduced into a specific site. This technique can be carried out in accordance with, for example, Molecular Cloning, A laboratory Manual, 2nd Ed., Cold Spring Harbor Laboratory, Cold Spring Harbor, NY, 1989; Current Protocols in Molecular Biology, Supplements 1-38, John Wiley & Sons (1987-1997).

The aforementioned "nucleotide sequence being hybridized under stringent conditions" refers to a nucleotide sequence of DNA obtained by colony hybridization, plaque hybridization, Southern hybridization, or the like using DNA as a probe. For instance, an example of the DNA used is DNA that can be identified by carrying out hybridization at 65°C in the presence of 0.7 to 1.0 M NaCl using a filter in which DNA derived from a colony or plaque or a fragment thereof is immobilized, followed by washing of the filter at 65°C using 0.1 to 2 × SSC solution (1 × SSC solution comprises 150 mM sodium chloride and 15 mM sodium citrate). Hybridization can be carried out in accordance with methods described in Molecular Cloning: A laboratory Manual, 2nd Ed., Cold Spring Harbor Laboratory, Cold Spring Harbor, NY, 1989.

An example of DNA hybridized under stringent conditions is DNA having a certain level or more of homology to the nucleotide sequence of DNA used as a probe. For instance, such DNA has a homology of 70% or more, preferably 80% or more, more preferably 90% or more, further preferably 93% or more, and particularly preferably 95% or more to the DNA used as a probe.

The aforementioned "gene having a nucleotide sequence encoding a protein having cancer-suppressing activity, such nucleotide sequence being hybridized with the nucleotide sequence set forth in SEQ ID NO: 1 under stringent conditions" can be obtained as described above by colony hybridization, plaque hybridization, or Southern hybridization under certain hybridization conditions.

In accordance with the present invention, "homologous protein of NR1I2 protein" refers to: a protein having an amino acid sequence derived from the amino acid sequence set forth in SEQ ID NO: 2 by deletion, substitution, and/or insertion of one to several amino acids, the amino acid sequence having cancer-suppressing activity; or a protein having an amino acid sequence which is 70% or more homologous to the amino acid sequence set forth in SEQ ID NO: 2, the amino acid having cancer-suppressing activity.

Regarding the above "nucleotide sequence derived from the nucleotide sequence set forth in SEQ ID NO: 2 by deletion, substitution, or insertion of one or several amino acids," the range of "one to several amino acids" is not particularly limited. For instance, such description indicates 1 to 20 amino acids, preferably 1 to 10 amino acids, more preferably 1 to 7 amino acids, further preferably 1 to 5 amino acids, and particularly preferably 1 to 3 amino acids.

The above "amino acid sequence which is 70% or more homologous to the amino acid sequence set forth in SEQ ID NO: 2" indicates that such amino acid is at least 70% or more, preferably 80% or more, and more preferably 90% or more homologous to the amino acid sequence set forth in SEQ ID NO: 2.

NR1I2 protein may be a naturally occurring protein, a chemically synthesized protein, or a recombinant protein produced by gene recombination technology. In view of large scale production through relatively easy operations, a recombinant protein is preferable.

A naturally occurring protein can be isolated from a cell or tissue in which the protein has been expressed by an adequate combination of protein isolation methods. A chemically synthesized protein may be synthesized by chemical synthesis methods such as the Fmoc method (fluorenylmethyloxycarbonyl method) and the tBoc method (t-butyloxycarbonyl method). In addition, the protein of the present invention can be synthesized using a variety of commercially available peptide synthesis machines. A recombinant protein can be produced by introducing DNA having a nucleotide sequence encoding the protein (e.g., the nucleotide sequence set forth in SEQ ID NO: 1) into a suitable expression system.

In addition, a protein having an amino acid sequence derived from the amino acid sequence set forth in SEQ ID NO: 2 by deletion, substitution, or insertion of one to several amino acids or a protein having an amino acid sequence which is 70% or more homologous to the amino acid sequence set forth in SEQ ID NO: 2 can be adequately produced or obtained by persons skilled in the art based on the nucleotide sequence set forth in SEQ ID NO: 1, which is one example of the sequences of DNA encoding the amino acid sequence set forth in SEQ ID NO: 2.

A preferred embodiment of the cancer-suppressing agent of the present invention comprises, as an active ingredient, a recombinant vector which is obtained by incorporating the above NR1I2 gene or a homologous gene thereof into such vector. An example of the vector used is a virus vector or a vector for expression in animal cell.. Preferably, a virus vector is used.

Examples of such viral vector include a retroviral vector, an adenoviral vector, an adeno-associated viral vector, a baculovirus vector, a vaccinia vector, and lentiviral vector. Among them, a retroviral vector is particularly preferably used. This is because, after a viral vector infects with a cell, a virus genome is incorporated into a host chromosome such that a gene incorporated into the vector can be stably expressed for a long period of time.

As an example of a vector for expression in animal cell, pCXN2 (Gene, 108, 193-200, 1991), PAGE207 (JP Patent Publication (Kokai) No. 6-46841 (1994)), or a modified vector thereof may be used.

The above recombinant vector can be produced by introducing it into an adequate host for transformation and culturing the obtained transformant. When the recombinant vector is a viral vector, an animal cell capable of producing virus is used as a host into which the vector is introduced. Examples of such animal cell include COS-7 cell, CHO cell, BALB/3T3 cell, and HeLa cell. Examples of a host used for a retroviral vector include ψCRE, ψCRIP, and MLV. An example of a host used for an adenoviral vector and an adeno-associated viral vector is 293 cell derived from a human embryonic kidney. A viral vector can be introduced into an animal cell by a calcium phosphate method or the like. In addition, when a recombinant vector is a vector for expression in animal cell, as a host into which the vector is introduced, *Escherichia coli* K12, HB 101, DH5 α, or the like can be used. Transformation of *Escherichia coli* has been known to persons skilled in the art.

The obtained transformants are each cultured in an adequate medium under adequate conditions. For instance, a transformant of *Escherichia coli* can be cultured using a liquid medium (for example, pH 5 to 8) containing carbon sources, nitrogen sources, inorganic matter and the like, which are necessary for cell growth. In general, culture is carried out at 15°C to 43°C for about 8 to 24 hours. In such case, a recombinant vector of interest can be obtained after the termination of culture by general DNA isolation methods.

Further, transformants of animal cells can be cultured using media such as 199 medium, MEM medium, and DMEM medium containing about 5% to 20% of bovine fetal serum. The pH of the medium is preferably about 6 to 8. In general, culture is carried out at about 30°C to 40°C for about 18 to 60 hours. In such case, since viral particles containing a recombinant vector are dispersed into a culture supernatant, a recombinant vector of interest can be obtained as a result of concentration and purification of viral particles by cesium chloride centrifugation, polyethylene glycol precipitation, and filter concentration.

As an example of the cancer-suppressing agent of the present invention, a cancer-suppressing agent (hereafter referred to as gene therapeutic agent) comprising as an active ingredient NR1I2 gene or a homologous gene thereof, can be produced by mixing NR1I2 gene or a homologous gene thereof as an active ingredient with a base generally used for a gene therapeutic agent. In addition, when NR1I2 gene or a homologous gene thereof is incorporated into a viral vector, viral particles containing a recombinant vector are prepared and the particles are mixed with a base generally used for a gene therapeutic agent.

As the above base, a base generally used for an injection can be used. Examples thereof include distilled water, a salt solution containing sodium chloride or a mixture of sodium chloride and an inorganic salt, a solution containing mannitol, lactose, dextran, glucose or the like, an amino acid solution of glycine, arginine or the like, and a mixed solution of an organic acid or salt solution and a glucose solution. Alternatively, in accordance with conventional techniques known by persons skilled in the art, using an adjuvant such as an osmoregulator, a pH adjuster, a plant oil, or a surfactant with such base, an injection can be prepared as a solution, suspension, or dispersion. Such injection can be prepared as a pharmaceutical that is solubilized at the time of use through operations such as pulverization and freeze drying.

In addition, the gene therapeutic agent of the present invention can be produced by adding NRI2 gene to a liposome suspension prepared in accordance with conventional techniques, followed by freezing and thawing. Liposomes can be prepared by filter penetration, ultrasonication, reverse phase evaporation, surfactant removal, or the like. Preferably, a gene is added to a liposome suspension that has been subjected to ultrasonication for reasons of the improved efficiency of gene encapsulation. A liposome encapsulating a gene can be intravenously administered alone or while suspended in water, saline, or the like.

The above gene therapeutic agent can be administered by general systematic administration through veins, arteries and the like, or local administration such as local injection or oral administration to a primary lesion of cancer or a predictable metastatic site. Further, administration of the gene therapeutic agent can also take place by a combination of catheterization, gene introduction, or surgical operations.

The dosage of the above gene therapeutic agent varies depending on patient's age, sex, and symptoms, the route of administration, the number of doses, and dosage forms. In general, the daily dosage (the weight of recombinant gene) ranges from 1 µg/kg body weight to 1000 mg/kg body weight for adults. Preferably, it ranges from 10 µg/kg body weight to 100 mg/kg body weight. The number of doses is not particularly limited.

Further, as an example of the cancer-suppressing agent of the present invention, a cancer-suppressing agent (hereafter referred to as protein formulation) comprising as an active ingredient NR1I2 protein or a homologous protein thereof is provided in the form of pharmaceutical composition comprising as an active ingredient NR1I2 protein or a homologous protein thereof and a pharmaceutical additive (e.g., a carrier or an excipient).

The form of the above protein formulation is not particularly limited. Examples of such form for oral administration include tablets, capsules, fine granules, powders, granules, liquids, and syrups. Examples of such form for parenteral administration include injections, infusions, suppositories, inhalants, transmucosa absorption systems, and transdermal absorption systems.

The route of administration of the above protein formulation is not particularly limited. The formulation can be administered by either oral administration or parenteral administration (e.g., intramuscular administration, intravenous administration, intradermal administration, transmucosa administration such as peritoneal administration, and inhalation administration).

The daily dosage of the above protein therapeutic agent varies depending on the patient's age, sex, symptoms, the route of administration, the number of doses, and the dosage form. In general, the daily dosage ranges from 0.001 µg/kg body weight to 1000 µg/kg body weight for adults. Preferably, it ranges from 0.001 µg/kg body weight to 100 µg/kg body weight. The number of doses is not particularly limited.

The above cancer-suppressing agent (including both forms of a gene therapeutic agent and a protein formulation) can be used for suppressing cancer by administering the effective dose thereof to mammalian animals including humans. The above cancer-suppressing agent can be used to prevent and/or treat cancer by administering the effective dose thereof for prevention and/or therapy to mammalian animals including humans.

The term "cancer-suppressing" herein has a broad meaning to a maximum extent, including preventive effects of preventing development, metastasis and implantation of cancer and therapeutic effects of inhibiting cancer cell growth, halting progression of cancer by reducing the size of cancer, and improving symptoms. In any case, it should not be interpreted in a limited manner.

Examples of cancer to be treated with the cancer-suppressing agent of the present invention include, but are not limited to, malignant melanoma, malignant lymphoma, lung cancer, esophageal cancer, gastric cancer, large-bowel cancer, rectal cancer, colon cancer, urinary tract tumor, gallbladder cancer, bile duct cancer, biliary tract, breast cancer, liver cancer, pancreatic cancer, testis tumor, maxillary cancer, tongue cancer, lip cancer, oral cavity cancer, pharynx cancer, larynx cancer, ovarian cancer, uterine cancer, prostate cancer, thyroid gland cancer, brain tumor, Kaposi's sarcoma, angioma, leukemia, polycythemia vera, neuroblastoma, retinoblastoma, myeloma, bladder tumor, sarcoma, osteosarcoma, myosarcoma, skin cancer, basal-cell carcinoma, cutaneous appendage tumor, skin metastatic cancer, and skin melanoma. In addition, among these, neuroblastoma is particularly preferable as a target of treatment.

### (2) Method for detecting cancer using an NR1I2 gene

The method for detecting and diagnosing cancer of the present invention comprises the step of analyzing NR1I2 gene in a test sample using DNA or RNA containing NR1I2 gene in its entirety or a part thereof. The method for detecting and diagnosing cancer of the present invention may be a method for detection used to screen for cancer to which the cancer-suppressing agent of the present invention is applied.

Herein, a part of NR1I2 gene is, for example, an oligonucleotide having a nucleotide sequence comprising about 10 to 30 consecutive nucleotides constituting the nucleotide sequence of the NR1I2 gene set forth in SEQ ID NO: 1. Examples of a test sample that can be used include tissue section, blood, lympha, sputum, lung lavage liquid, urine, feces, and tissue culture supernatant, which are suspected to contain the presence of tumor.

The above expression "detection to screen for cancer to which the cancer-suppressing agent of the present invention is applied" indicates discovering the presence or absence of cancer in tissue or the like, on which the cancer-suppressing agent of the present invention effectively acts.

Detection of cancer to be screened for is carried out by analyzing NR1I2 gene in a test sample using DNA or RNA containing NR1I2 gene in its entirety or a part thereof as a primer or probe. Specifically, the expression "analyzing NR1I2 gene" used herein indicates detection of methylated genomic DNA or detection of an abnormal level of gene expression.

Detection of gene mutation is carried out as follows. When the above DNA or RNA is used as a primer, a partial sequence of DNA prepared from a test sample is amplified by PCR using, for example, selected two types of primers having different sequences, followed by treatment with sodium hydrogen sulfite. Thus, unmethylated cytosine (C) in the genomic DNA is converted into uracil (U). Since methylated cytosine is structurally stable, it does not undergo such reactions and is not converted into uracil. The presence of methylated DNA can be confirmed by analyzing unmethylated cytosine and cytosine that has been modified as a result of being combined with a restriction enzyme susceptive to the influence of methylation, after being subjected to restriction enzyme cleavage (COBRA). Alternatively, such detection can be carried out by: amplifying a partial sequence of DNA prepared from a test sample by PCR; allowing the amplified product to be subjected to treatment with sodium hydrogen sulfite; incorporating the amplified product into a plasmid; transforming a host cell with it and culturing the transformant; and analyzing the nucleotide sequence of the clone obtained.

Meanwhile, detection of abnormal levels of gene expression can be carried out by Northern hybridization or RT-PCR (reverse transcription-polymerase chain reaction) using a probe containing the above RNA sequence.

### (3) Method for detection to screen for cancer using NR1I2 protein antibody or a fragment thereof.

The method for detecting and diagnosing cancer of the present invention comprises a step of analyzing the amount of NR1I2 proteins in a test sample using an antibody against NR1I2 protein or a fragment thereof. The method for detecting and diagnosing cancer of the present invention may be a method for detection to screen for cancer to which the cancer-suppressing agent of the present invention is applied.

The antibody against the NR1I2 protein used in the present invention (hereafter referred to as NR1I2 antibody) can be produced by a conventional method using NR1I2 protein in its entirety or a part thereof as an antigen. The term "a part of an NR1I2 protein" indicates a polypeptide comprising at least 6 amino acids, preferably about 8 to 10 amino acids, and further preferably about 11 to 20 amino acids, which are consecutive amino acids constituting the amino acid sequence of the NR1I2 protein set forth in SEQ ID NO: 2. The NR1I2 protein in its entirety or a part thereof serving as an antigen may be prepared by either biological or chemical techniques.

A polyclonal antibody can be prepared in the following manner: the above antigen, for example, is repeatedly used for subcutaneous, intramuscular, intraperitoneal, and intravenous inoculation in animals such as mice, guinea pigs, and rabbits such that the animals are sufficiently immunized; blood is collected from the animals; and serum separation is performed. A monoclonal antibody can be prepared from a culture supernatant of a hybridoma or ascites of a mouse into which the hybridoma has been administered, such hybridoma being obtained by cell fusion of commercially available mouse myeloma cells and splenic cells of a mouse immunized with, for example, the above antigen.

It is possible to measure the expression level of NR1I2 protein in a test sample using NR1I2 protein antibody or a fragment thereof prepared as described above. For instance, the measurement of the expression level can be carried out using Western blotting or immunological methods such as immunoblotting, enzymeimmunoassay (EIA), radioimmunoassay (RIA), a fluorescent antibody method, and immunocyto-staining. Herein, a fragment of NR1I2 protein antibody refers to a single-chain fragment variable (scFv) of an antibody of interest or the like. In addition, examples of a test sample that can be used include tissue section, blood, lympha, sputum, alveolar lavage liquid, urine, feces, and tissue culture supernatant, which are suspected to exhibit the presence of tumor. The low expression level of NR1I2 protein in a test sample subjected to measurement indicates that the expression of the NR1I2 gene is suppressed in the sample tissue or cells. Therefore, cancer to which the cancer-suppressing agent of the present invention is applied can be screened for.

The present invention is hereafter described in greater detail with reference to the following examples, but the technical scope of the present invention is not limited thereto.

### Examples

### Example 1: Isolation of methylated DNA from neuroblastoma by BAMCA

BAMCA was carried out using MCG Whole Genome Array-4500 (Inazawa J., et al., Cancer Sci. 95 (7), 559, 2004). Neuroblastoma-cultured cell lines IMR32 and GOTO were used as test samples, and a sample containing DNA taken from 5 patients at neuroblastoma stage 1 was used as a control sample. Table 1 lists BAC clones and genes contained therein. These BAC clones were isolated as methylated DNA having a value of 1.5 or more. Such value was obtained by dividing fluorescent signals of IMR32 and GOTO cells labeled with Cy3 with a fluorescent signal of a mixture of cancer cells at stage 1 labeled with Cy5 as a control sample. NR1I2 gene is contained in a BAC clone (No. 169N13), and it contains DNA having a CpG island. Further, upon measurement of the amount of messenger RNA of NR1I2 gene, the expression of messenger RNA was found in the control sample; however, it was not found in the IMR32 and GOTO cells. It is possible to decrease the level of DNA methylation after continuously culturing cells with the addition of 5-aza-deoxycytidine (5-aza-dCyd), which is an inhibitor of DNA methyltransferase. Thus, a GOTO cell was cultured in a medium containing 5-aza-dCyd at a concentration of 1 µM for 5 days, followed by measurement of the amount of messenger RNA. As a result, the expression of NR1I2 gene was found to have recovered. This indicates that DNA in an NR1I2 gene is usually methylated in neuroblastoma GOTO cells so that the expression level of messenger RNA is decreased, and that the messenger RNA expression is induced upon demethylation. In other words, NR1I2 gene was found to be a cancer-suppressing gene in which the expression of messenger RNA is controlled in advanced neuroblastoma due to DNA methylation.

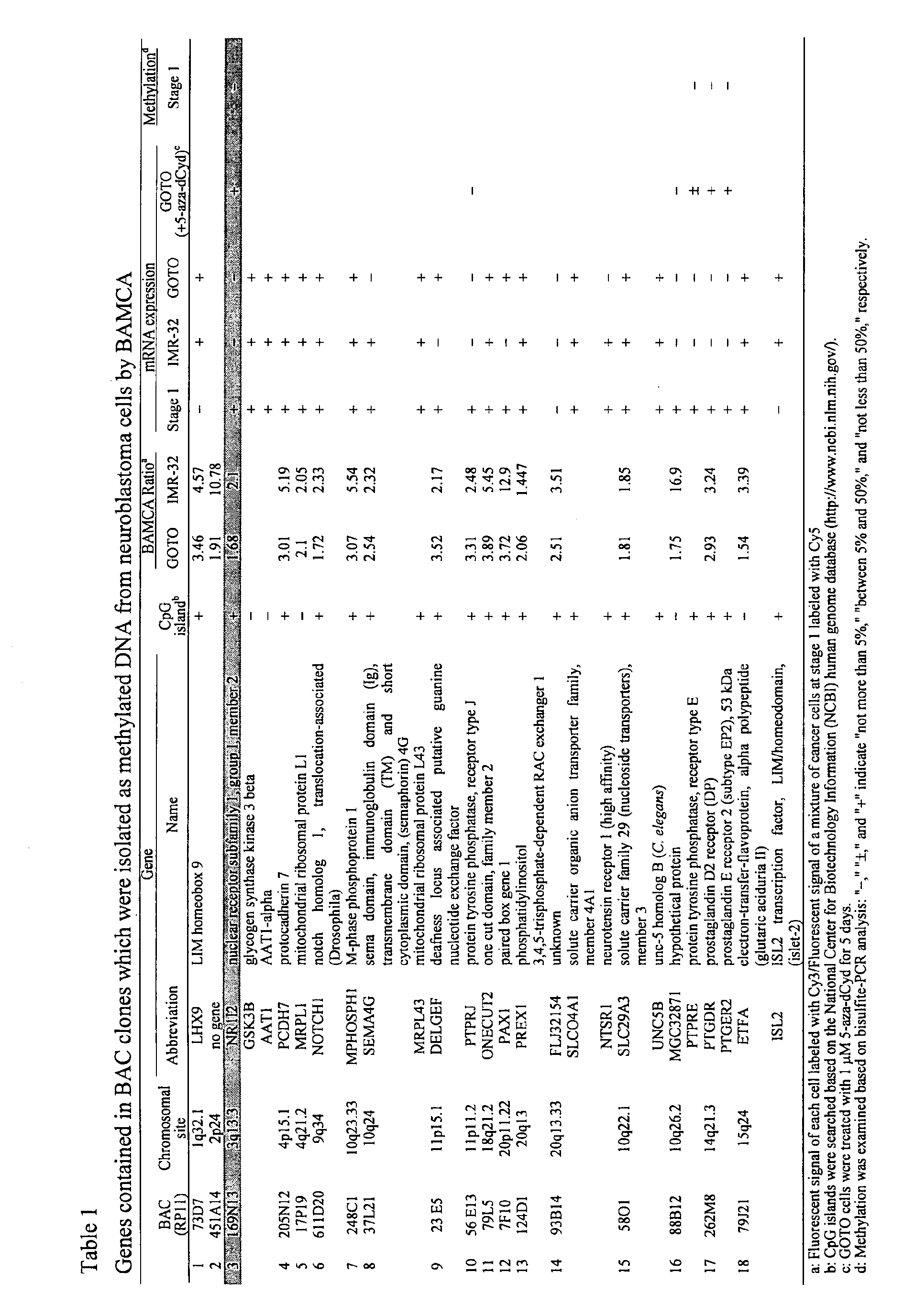

### Example 2: Structure of a BAC clone 169N13 containing NR1I2 gene

Fig. 1 shows the structure of NR1I2 gene in the BAC clone 169N13 containing methylated DNA, which was obtained using a neuroblastoma cultured cell line. The genomic DNA structure of NR1I2 gene contains 9 exons and CpG-rich regions designated as Regions 1 and 2. Region 1 is located upstream of the exons, and Region 2 comprising 39 nucleotides in present in exon 3.

### Example 3: Confirmation of methylation frequency in CpG regions of NR1I2 gene

IMR32 and SH-SY5Y cells lacking the expression of messenger RNA of NR1I2 gene, and SK-N-AS, SJ-N-KP, and LCL cells having the expression of messenger RNA of NR1I2 gene were used. Genomic DNAs of these cells were treated with sodium hydrogen sulfite such that unmethylated cytosine (C) in each genomic DNA was converted into uracil (U). Thereafter, CpG regions of NR1I2 gene were amplified by PCR, and were incorporated into a plasmid so as to confirm nucleotide sequences of the CpG regions. The nucleotide sequence of each cell was verified 3 to 5 times. In Fig. 2, methylated and unmethylated CpG regions of the nucleotides are shown in black and gray, respectively. It has been revealed that there is an inverse correlation between the expression of messenger RNA in NRI2 gene (expression: "+" and "-") and methylation in Region 2.

### Example 4: Cell culture

Cell lines used were human-derived neuroblastoma cells SK-N-KS, SK-N-AS, SK-N-SH, SK-N-DZ, SH-SY5Y, MP-N-TS, MP-N-MS, KP-N-RT, KP-N-SIFA, KP-N-SILA, KP-N-TK, KP-N-YS, SMS-KCN, SMS-KAN, SJ-N-CG, NB-1, CHP134, IMR32, and GOTO (Saito-Ohara F, et al. Cancer Res., 63, 1876, 2003), which were derived from cervical cancer HeLa cells and surgically excised samples. In addition, in order to obtain a material subjected to inhibition of DNA methylation, 5-aza-deoxycytidine (5-aza-dCyd) serving a DNA methyltransferase inhibitor was added to each cell at a concentration of 1 µM for 5 days during culture.

### Example 5: Messenger RNA expression of NR1I2 gene

In order to measure the expression level of messenger RNA of NR1I2 gene, 15 neuroblastoma cell lines regarding each of which the presence or absence of N-myc gene amplification had been known were subjected to RT-PCR using an adrenal gland (Adr-gland) as a control (fig. 3). As a control for the expression level obtained by RT-PCR, GAPDH (glyceraldehyde-3-phosphate-dehydrogenase) was used because it had been known that the expression level of GAPDH was unlikely to vary depending on cell species and conditions. As a result, it was revealed that there was a correlation between N-myc gene amplification and a phenomenon of the absence of the expression of messenger RNA of NR1I2 gene. Based on advanced malignancy in neuroblastoma along with degree of N-myc gene amplification, it has been discovered that the lowered expression level of messenger RNA of NR1I2 gene is involved in malignant development of neuroblastoma.

### Example 6: Comparison between the expression levels of messenger RNA in methylated and demethylated NR1I2 genes

Under general culture conditions, 8 neuroblastoma cell lines lacking the expression of messenger RNA of NR1I2 gene were cultured with the addition of 5-aza-deoxycytidine (5-aza-dCyd) serving as a DNA methyltransferase inhibitor at a concentration of 1 µM for 5 days. The expression levels of messenger RNA of NR1I2 gene in the cells were compared with each other by RT-PCR (fig. 4). As a control for the expression level obtained by RT-PCR, GAPDH was used. In all cell lines tested, the expression of messenger RNA of NR1I2 genes was observed as a result of inhibition of methylation. Thus, it has been revealed that the expression of NR1I2 gene expression is controlled by the methylation of its genomic DNA.

### Example 7: Correlation between gene expression and methylation detection in Region 2 of NR1I2 gene by COBRA

B lymphoma cell line (LCL) in which the expression of NR1I2 gene had been confirmed and 11 neuroblastoma cell lines were subjected to COBRA so as to detect methylation in Region 2 of the NR1I2 gene (fig. 5). Fig. 5 shows that cell lines exhibiting a band at M position have methylated Regions 2. There is a good correlation between cell lines having methylated Regions 2 and the absence of the NR1I2 gene expression. That is, it has been revealed that the NR1I2 gene expression is negatively regulated by methylation of Region 2 of NR1I2 gene.

### Example 8: Detection of methylation frequency in Region 2 of NR1I2 gene by COBRA method using clinical samples

Table 2 shows results of analysis of methylation in Region 2 of an NR1I2 gene. Samples used were 51 neuroblastoma samples which had been excised at Kyoto Prefectural University of Medicine from 1986 to 2003 with parental consent and the approval of the ethics committee. Among the samples, 12 cases were at stage 1, 11 cases were at stage 2, 8 cases were at stage 3, 13 cases were at stage 4, and 4 cases were at stage 4s. Also, 37 cases were obtained from infant patients under 1 year old. N-myc amplification was observed in 8 out of 51 cases (15%), and 39 cases (76%) were found through neuroblastoma mass screening.

P-values were calculated using the Fisher's exact test. The obtained values considered significant are shown in bold. Staging was based on the International Neuroblastoma Staging System (INSS) (Brodeur GM, et al., J Clin Oncol, 11,1466,1993).

The analysis has revealed that there is a correlation between methylation in Region 2 of NR1I2 gene and stage progression, N-myc amplification and poor prognosis. The results indicate that methylation in Region 2 of NR1I2 gene is significantly involved in malignant alteration of neuroblastoma. Thus, it has been revealed that methylation in Region 2 of NR1I2 gene and the decreased expression level of NR1I2 gene play an important role in malignant alteration of neuroblastoma.

**Table 2: Detection of methylation frequency in Region 2 of NR1I2 gene using 51 neuroblastoma samples**

| Items | | Number of cases | Methylation in Region 2 of NR1I2 gene | | |
|---|---|---|---|---|---|
| | | | Negative | Positive | P-value |
| Total | | 51 | 42 | 9 | |
| Age | Under 1 year old | 33 | 30 | 3 | 0.052 |
| | Aged 1 year and older | 18 | 12 | 6 | |
| Stage | I, II, IVs | 30 | 23 | 2 | **0.0234** |
| | III, IVa | 21 | 16 | 7 | |
| N-myc | Amplification | 43 | 39 | 4 | **0.024** |
| | No amplification | 8 | 3 | 5 | |
| Prognosis | Alive | 44 | 39 | 5 | **0.0135** |
| | Dead | 7 | 3 | 4 | |

### Example 9: Measurement of transcriptional activation effects of Regions 1 and 2 of NR1I2 gene by reporter assay

With the use of Region 1 comprising 1060 nucleotides located upstream of exon 1 of NR1I2 gene and Region 2 comprising 480 nucleotides and containing exon 3, the presence or absence of transcriptional activation was tested by reporter assay. The reporter assay system used was based on a method for measuring a light-emitting substance, which is generated as a function of an enzyme, such enzyme being converted into a protein as a result of the expression of messenger RNA of firefly luciferase. Constructs of genes were obtained by preparing genes in a manner such that a luciferase gene was disposed in the center using pGL3-Basic vector (Promega) and Regions 1 and 2 of an NR1I2 gene were combined as shown in Fig. 6A. The genes were introduced into HeLa and SK-N-AS cells, followed by measurement of the luciferase activity after 36 hours. In order to correct a difference in terms of the efficiency of gene introduction, pRL-hTK (a vector that corrects the expression level of an experimental firefly luciferase reporter gene using the expression level of Renilla luciferase as an internal control; Promega) was used. Then, the luciferase activity of each gene obtained as a relative value was determined to be the transcriptional activity of the gene. Mock is a negative control used upon gene introduction using a pGL3-Basic vector. Fig. 6B shows results of measurement of luciferase activities of 1/L and 2/L in HeLa and SK-N-AS cells. In both cases, cells into which a 2/L gene had been introduced were found to have high levels of luciferase activity. The results indicate that Region 2 has a high degree of ability to cause transcriptional activation. Meanwhile, CpG-rich Region 1 was found to have little ability to cause transcriptional activation, although it was considered to be a region for transcriptional regulation of the NR1I2 gene. Next, Fig. 6C shows results of measurement of luciferase activity in the manner described above after Mock, 1/L, 2/L, L/2, and 1/L/2 genes were introduced into SK-N-AS cells. As with the case of Fig. 6B, Fig. 6C indicates that cells into which 2/L had been introduced showed high levels of luciferase activity. On the other hand, in the case of L/2, the activity level was at half of that of 2/L, and in the case of 1/L/2, the level of transcriptional ability was almost equivalent to that of the negative control. The results indicate that Region 2 of the NR1I2 gene has a stable ability to cause transcriptional activation and the ability is exerted upstream of the transcription initiation site. With regard to 1/L/2 having little ability to cause transcriptional activation to a luciferase gene, it was found that Region 1 inhibited the ability of transcriptional activation, the level of which had been elevated in the case of L/2.

### Example 10: Relationship between detection of methylation in Region 2 of NR1I2 gene in a clinical sample by COBRA method and NR1I2 gene expression, N-myc gene amplification, stage and prognosis

Detection of methylation in Region 2 of NR1I2 gene was carried out using samples. The samples used were 51 neuroblastoma samples which had been excised at Kyoto Prefectural University of Medicine from 1986 to 2003 with parental consent and the approval of the ethics committee.

Fig. 7A shows results of detection of methylation in Region 2 of the NR1I2 gene by the COBRA method using the following representative 9 samples selected among samples subjected to stage classification: T597, T399, T974, T621, T491, T4773, T304, T530, and T5718. Fig. 7A shows that samples exhibiting a band at M position have methylated Regions 2. Likewise, Fig. 7B shows results of RT-PCR that was conducted to confirm the expression of messenger RNA of NR1I2 gene using RNAs obtained from the above 9 samples. In order to obtain a control of the expression level, GAPDH was used. Figs. 7A and 7B have revealed that, as with the case of samples using cells, there is a negative correlation between methylation in Region 2 and the expression of messenger RNA in NRI2 gene in the case of clinical samples.

In 47 out of 51 cases of the neuroblastoma samples, high-quality messenger RNA was obtained. Thus, the expression level of messenger RNA of NR1I2 gene was measured by RT-PCR and the amount of messenger RNA in GAPDH was measured as a control. Then, the relative ratio therebetween was calculated. Fig. 7C shows graphs in which vertical axes indicate the relative ratio, regarding the following four items

the presence or absence of methylation in Region 2 of NR1I2 gene (measured by COBRA method);
the presence or absence ofN-myc gene amplification;
neuroblastoma stage; and
prognostic outcome (alive or dead).
Except for the graph of prognostic outcome, it was confirmed that the expression level of messenger RNA of NR1I2 gene was significant (Mann-Whitney U test). That is, with the use of clinical neuroblastoma samples, it has been revealed that:
there is an inverse correlation between methylation in Region 2 of NR1I2 gene and the expression of messenger RNA;
samples having N-myc gene amplification have low expression levels of NR1I2 messenger RNA; and
the expression level of NR1I2 messenger RNA decreases as neuroblastoma progresses.

### Example 11: Decrease of carcinogenicity due to NR1I2 gene introduction into neuroblastoma cells

Adherence to solid matter has been known as an example of a difference between properties of normal cells and those of cancer cells under culture conditions,. In other words, it is necessary for normal cells to adhere to solid matter for cell proliferation (anchorage dependence). When normal cells are cultured in a soft agar medium (about 0.33%), they can not proliferate while floating in such medium. However, many cancer cells lack anchorage dependence so that they can proliferate in a soft agar medium. Since floating cancer cells cannot move in a soft agar medium, a single cancer cell is repeatedly divided such that the divided cells form a colony in 1 to 3 weeks time. Since there is a correlation between the ability of the cell to form a soft agar colony and the implanted tumor forming ability, the degree of such ability has been widely used as an index of malignancy (Shin, S. I., et al., Proc. Natl. Acad. Sci. USA 72, 4435, 1975). A full-length NR1I2 cDNA was inserted into pCMV-Tag3 vector (Stratagene) in which an myc peptide can be added to the amino terminal of an inserted gene (pCMV-Tag3-NR1I2). In addition, we already have discovered that a fusion product of a herpes-virus-derived VP16 protein and an NR1I2 protein has stronger transcriptional activity than that of an NR1I2 protein alone. Thus, VP16 cDNA and a full-length NR1I2 cDNA were inserted into pCMV-Tag3 vector (pCMV-Tag3-VP-NR1I2). IMR32 and SMS-KAN cells obtained from neuroblastoma cell lines lacking NR1I2 gene expression were subjected to gene introduction using Fugene6 (Roche Diagnostics). Culture was carried out for 3 weeks in the presence of 500 µg/ml of G418 (Geneticin), followed by crystal violet staining. Then, the number of colonies was counted (Figs. 8A and 8B). In the cases of both IMR32 and SMS-KAN cells, it was confirmed that the number of colonies was significantly reduced by NR1I2 gene expression. Particularly, when using pCMV-Tag3-VP-NR1I2, the number of colonies was significantly reduced. This phenomenon indicates that the NR1I2 gene has a function of suppressing cancer cell growth. Thus, the gene has been found to function as a cancer-suppressing gene in neuroblastoma.

### Example 12: Decrease of carcinogenicity due to NR1I2 gene introduction into neuroblastoma cells

pCMV-Tag3 vectors not containing an inserted gene, and pCMV-Tag3-VP-NR1I2 were introduced into SMS-KAN cells, and thus one type of control cell clone (empty) and two types of cell clones permanently expressing NR1I2 proteins (B1 and B2) were obtained. Disrupted cell solutions of these three types of cells were subjected to Western blotting, followed by detection using an antibody against myc protein. The results are shown in Fig. 9A. Compared with the control cell (empty), NR1I2 proteins were found to be expressed in the other cells. Fig. 9B shows the growth rates of the cells. The numbers of growing cells were monitored by utilizing conversion of tetrazolium salt (WST-1) into formazan dye due to a function of mitochondrial dehydrogenase in a viable cell (using a cell counting kit-8, Dojindo Laboratories). Since NR1I2 proteins were expressed in both clones B 1 and B2, the degrees of cell growth therein were significantly reduced after day 3 of culture compared with the case of the control cell (empty). (In the Figure, "a" and "b" indicate a P-value in the Mann-Whitney U test of less than 0.05 in the cases of control cells (empty) compared with clone B1 and clone B2, respectively.)

Based on these results, it has been revealed that cell growth is suppressed due to a function of the NR1I2 protein expressed in a cell. Since known cancer-suppressing gene proteins suppress cell growth and induce cell death so as to protect a living body from cancer, the NR1I2 protein has been found to have an effect of product of cancer-suppressing gene.

### Example 13: Search of a gene controlled by NR1I2 gene

The NR1I2 gene has been known as a transcriptional factor. Thus, it is possible to know what type of gene expression is controlled by obtaining messenger RNAs from cells causing NR1I2 protein expression and cells not causing such expression, and comparing the expression levels thereof with each other. As described above, the NR1I2 gene protein may have an effect of inhibiting cancer. It is considered that such effect is actually exhibited by a gene product controlled by the NR1I2 gene protein. Therefore, pCMV-Tag3 vector not containing a foreign gene as a control was introduced into SMS-KAN cell, and pCMV-Tag3-VP-NRI12 containing the NR1I2 gene as a test sample was introduced into SMS-KAN cell. As a result, a control cell clone and a cell clone B1 permanently causing NR1I2 protein expression were obtained. Thereafter, analysis of messenger RNA expression was carried out using these two types of cells and an AceGene Human oligo chip 30 K (DNA Chip Research Inc.), with which the expression levels of 30,000 human genes can be observed (Inoue. J., et al. Am J Pathol. 165. 71. 2004). A complementary strand DNA (complimentary DNA) was prepared, into which aminoallyl-dUTP (Ambion Inc.) was incorporated using an Oligo (dT) 12-18 primer. Then, the aminoallyl group was labeled with reactive Cy3 (cyanin 3) and Cy5 (cyanin 5) (Amersham Biosciences) via a coupling reaction. Hybridization was performed twice (1^{st}: Cy3-B1/Cy5-control; 2^{nd}: Cy5-B1/Cy3-control). Signals were measured using a GenePix 4000B (Axon Instruments) so as to be analyzed by GenePix Pro 4.1 software (Axon Instruments). Tables 3 and 4 are lists of genes, where in the cases of SMS-KAN cells into which the NR1I2 gene had been introduced in the two experiments, the expression levels were 1.5-fold or greater than the expression level in the case of a control cell clone.

Since the experiment results involved the CYP3A4 gene, the expression level of which had been known to increase due to the existence of NR1I2 gene, the experiment was confirmed to be reliable.

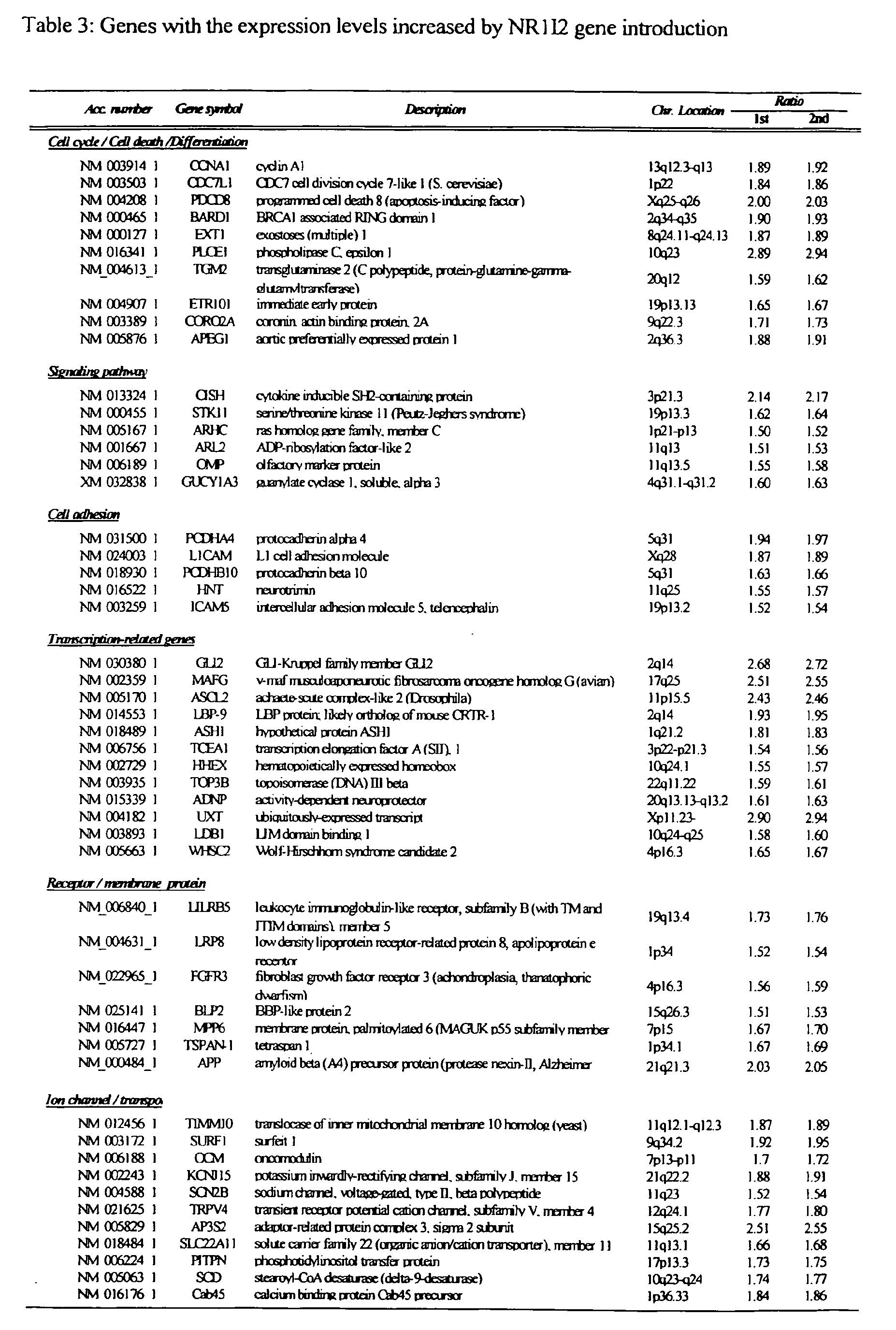

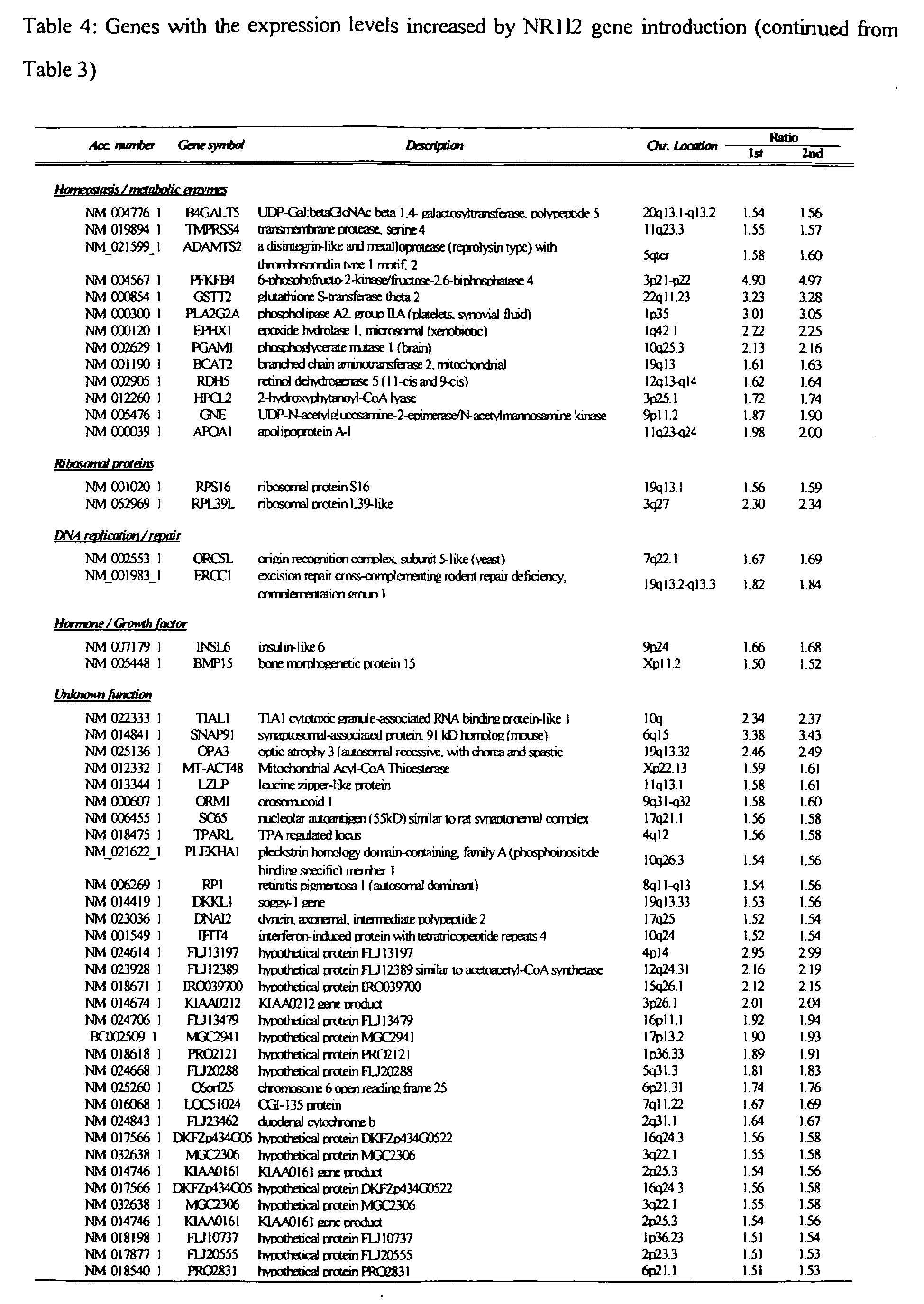

### Example 14: Genes expected to have a cancer-suppressing effect which are controlled by the NR1I2 gene

It has been known that NR1I2 gene is believed to have a function of inducing messenger RNA of an enzyme group that detoxifies a foreign substance upon the invasion of a living body by such substance (Blumberg B, et al. Genes Dev, 12, 3195, 1998; Kliewer SA, et al. Cell, 92, 73, 1998; and Xie W, et al. Nature, 406, 435, 2000). For instance, NR1I2 protein induces messenger RNA of CYP3A4, which is an enzyme metabolizing a foreign substance, and also induces messenger RNA ABCB 1 (P-glycoprotein), which is an elimination pump for an intracellular drug (Synold TW, et al. Nat Med, 7, 584, 2001). However, no genes have been reported to be involved in cancer suppression controlled by NR1I2 gene. Thus, 10 types of genes were selected as candidate genes involved in cancer growth inhibition from among the 105 types of genes obtained by the above experiment of transcriptional analysis based on existing literature information and OMIM information (Online Mendelian Inheritance in Man, http://www.ncbi.nlm.nih.gov/Omim/omimhelp.html). Then, the expression levels of the genes were observed by RT-PCR with electrophoresis (Fig. 10).

The messenger RNA materials used were obtained from (A) SMS-KAN as a control, (B) KAN-NR1I2 expression clone B1, (C) KAN-NR1I2 expression clone B2, (D) GOTO as a control, and (E) GOTO-NR1I2 expression clone A1. DNA generated by PCR was subjected to electrophoresis using 3% agar gel, followed by quantification of bands using LAS-3000 (Fujifilm). The GAPDH gene was subjected to a similar experiment and the result was corrected as a control value. Then, the expression level of each gene was calculated as a relative value with respect to the control value. It was considered that a gene with an expression level that increased in correlation with NR1I2 gene expression was under the control of the NR1I2 gene. The strongest correlation was between the NR1I2 gene and the PLA2G2A gene. Fig. 11 shows actual electrophoresis images. It was confirmed that there was a good correlation between NR1I2 gene expression and PLA2G2A gene expression.

The PLA2G2A gene has been known to be related to secretory phospholipase A2. It is remarkable that the chromosomal site at which a PLA2G2A gene exists is site 1p36, deletion of which is often observed in neuroblastoma (Praml C, et al. Cancer Res, 55, 5504, 1995). In addition, it has been reported that the number of colonic polyps increases in a PLA2G2A gene knockout mouse during experimentation. Thus, the PLA2G2A gene has been found to be associated with colon cancer (Haluska FG, et al. Int J Cancer 72, 337, 1997).

### Conclusions of Examples

(1) Based on screening by the BAMCA method, NR1I2 gene was isolated, in which genomic DNA had a hypermethylated site, and of which messenger RNA was expressed at a lowered level in malignant neuroblastoma. Further, as a result of an experiment with the use of cells derived from clinical samples, it has been found that the decreased level of NR1I2 gene expression can be improved upon demethylation.
(2) It has been revealed that Region 2 (the exon 3 region) of NR1I2 gene has ability to cause transcriptional activation and methylation in Region 2 contributes to the decreased expression level of messenger RNA. Based on analysis of clinical samples, there is a correlation between methylation in Region 2 and neuroblastoma stage progression, N-myc gene amplification, and poor prognosis. Moreover, it has also been revealed that there is an inverse correlation between a high expression level of the NR1I2 gene, methylation in Region 2, neuroblastoma stage progression, and N-myc gene amplification.
(3) After NR1I2 gene was introduced into a neuroblastoma cell line in which NR1I2 gene was not expressed, the degree and the rate of cell growth decreased during an experiment of anchorage dependence. Thus, it has been revealed that, when NR1I2 gene has been expressed as a protein in a cancer cell, such cell loses its properties as cancer cells; that is to say, the NR1I2 gene functions as an cancer-suppressing gene.
(4) NR1I2 gene has been reported to function as a transcriptional factor; and has a function of controlling the expression of other genes. Thus, the NR1I2 gene was introduced into a neuroblastoma cell line in which such gene had not been expressed. Then, genes with increased expression levels were screened for. As a result, PLA2G2A has been found as a gene controlling cell growth under the control of NR1I2 gene.

### Effects of the Invention

In accordance with the present invention, there is provided a cancer-suppressing agent which comprised NR1I2 gene, which has been newly found to have a function of suppressing cancer, or NR1I2 protein encoded by this gene is provided. These agents are very useful in view of clinical applications such as the treatment based on individual differences of cancers and the improvement of cancer prognosis, or in view of basic cancer research.

## Claims

1. A cancer-suppressing agent which comprises NR1I2 gene or a homologous gene thereof

2. The cancer-suppressing agent of claim 1, wherein the gene or a homologous gene thereof is incorporated into a vector.

3. The cancer-suppressing agent of claim 2, wherein the vector is a viral vector or plasmid vector for expression in animal cell.

4. The cancer-suppressing agent of claim 3, wherein the viral vector is a retroviral vector, adenoviral vector, adeno-associated viral vector, baculovirus vector, vaccinia vector, or lentiviral vector.

5. The cancer-suppressing agent of claim 1, wherein the gene or a homologous gene thereof is encapsulated in a liposome.

6. A cancer-suppressing agent which comprises NR1I2 protein or a homologous protein thereof.

7. A method for detecting and diagnosing cancer, which comprises a step of analyzing NR1I2 gene in a test sample using DNA or RNA containing NR112 gene in its entirety or a part thereof.

8. The method of claim 7, wherein the analysis involves detection of mutation of the gene or detection of abnormal expression level of the gene.

9. The method of claim 7, which is a method for screening for cancer to which the cancer-suppressing agent of claim 1 can be applied.

10. A method for detecting and diagnosing cancer, which comprises a step of analyzing NR1I2 protein in a test sample using an antibody against NR1I2 protein or fragment thereof.

11. The method of claim 10, wherein the analysis involves detection of abnormal expression level of the protein.

12. The method of claim 10, which is a method for screening for cancer to which the cancer-suppressing agent of claim 1 can be applied.
